# EUROPEAN PATENT SPECIFICATION

(11) **EP 2 559 449 B1**
(45) Date of publication and mention of the grant of the patent: **05.11.2014**
(21) Application number: 12174259.7
(22) Date of filing: 29.06.2012
(51) Int. Cl.: A61M 5/315, A61M 5/32, A61M 5/24, A61M 5/50

(54) **Safety syringe**
Sicherheitsspritze
Seringue de sécurité

(30) Priority: 06.07.2011 US 201161571794 P
(43) Date of publication of application: 20.02.2013
(73) Proprietor: King Saud University, 11421 Riyadh (SA)
(72) Inventor: Qureshi, Khurshid A., Colorado Springs, CO Colorado 80920 (US)
(74) Representative: Goddar, Heinz J.

(56) References cited:
- EP-A2- 0 272 035

## Description

### Field of the invention

The present invention relates to a safety syringe, and in particular to a disposable safety syringe for dental anesthesia and other medical applications.

### Background of the invention

Syringes are utilized for administering a drug. Known syringes are frequently pre-filled which is satisfactory for many medicaments. A problem with traditional syringes is the protection of the person handling the syringes and doing the injection. First, the medicament being injected can pose a risk for the medical personnel using the syringe, and further there is the risk of accidental pricking of the medical personnel.

EP 0 272 035 A2 discloses an injection device having a mechanism for retracting the needle inside a housing of the injection device after use.

### Summary of the invention

It is an object of the present invention to provide a simplified construction of a safety syringe which provides protection from accidental pricking of the person doing the injection or of other medical personnel handling the syringe.

According to the invention, there is provided a safety syringe, according to claim 1.

Preferably, a pushing force required to move the first engagement means distally relative to the cartridge is less than the minimum puling force, in particular less than 75 %, 50 %, 30 %, 20 % or 10 % thereof.

The plunger rod at its distal end may be provided with a second engagement means for cooperating with the piston, the second engagement means enabling the plunger rod to pull on the piston in a proximal direction for aspiration.

On the cartridge holder, a third locking means may be provided, and inside the housing a fourth locking means may be provided, the third locking means and the fourth locking means cooperatingly and automatically locking the cartridge holder upon movement of the cartridge holder into its proximal position.

Alternatively inside the housing a fourth locking means may be provided, the first locking means and the fourth locking means cooperatingly and automatically locking the cartridge holder upon movement of the cartridge holder into its proximal position.

The first engagement means may comprise a barbed design.

The second engagement means may comprise a screw thread and/or a hook.

### Brief description of the drawings

Having thus generally described the invention, reference will be made to the accompanying drawings illustrating an embodiment thereof, in which:
- Fig. 1: is a schematic side view of a cartridge holder;
- Fig. 2: is a schematic side view of a cartridge;
- Fig. 3: is a schematic side view of a syringe body;
- Fig. 4: is a schematic side view of a plunger;
- Fig. 5: illustrates a syringe assembled from the parts shown in Fig. 1 to Fig. 4;
- Fig. 6: illustrates an alternative embodiment of a plunger;
- Fig. 7: illustrates a syringe ready for injecting;
- Fig. 8: shows the syringe of Fig. 7 after completion of the injection; and
- Fig. 9: shows the syringe of Fig. 7, 8, the cartridge holder being in a retracted and locked, proximal position.

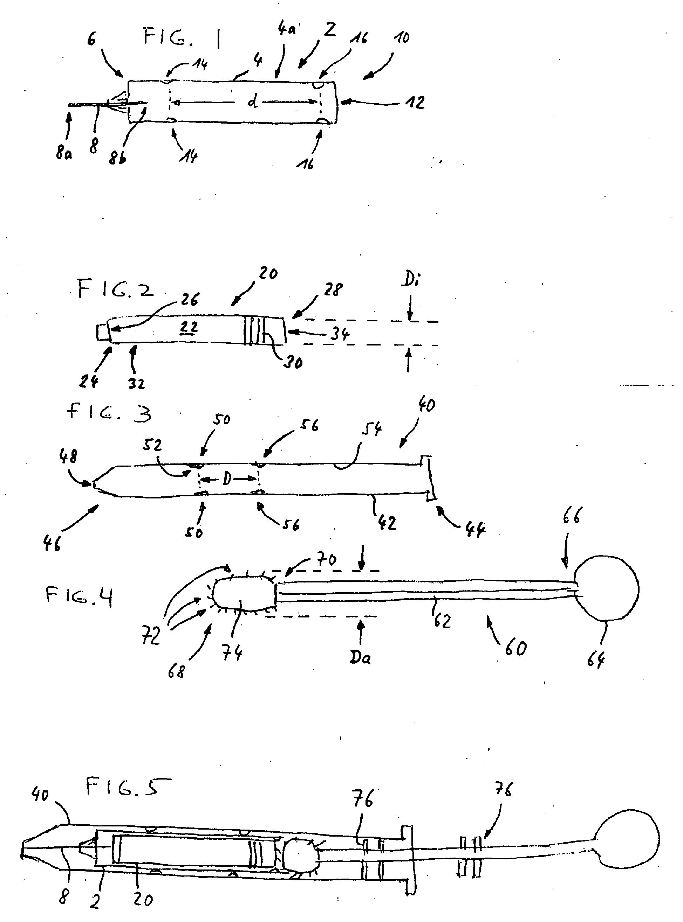

Fig. 1 to Fig. 4 show essential parts of a syringe according to the invention. A cartridge holder 2 shown in Fig. 1 has a cylindrical wall 4, a closed front or distal end 6 in which a needle 8 is fixed, and an open proximal end 10.

The needle 8 has a distal end 8a projecting distally from the distal end 6 of the cartridge holder 2, and a proximal end 8b projecting internally into internal space 12 enclosed by the cylindrical wall 4 of the cartridge holder 2.

The cylindrical wall 4 of the cartridge holder 2 is provided at its outside near to its distal end 6 with a first locking means 14. In the described embodiment, the first locking means 14 is formed by a circumferentially extending recessed portion in the cylindrical wall 4. Viewed in a longitudinal section, the recessed area has a circular cross section.

Further, the cartridge holder in the described embodiment is provided with a third locking means 16 spaced apart from first locking means 14 by a distance d. Similar to the first locking means 14, the third locking means 16 is formed by a circumferentially extending recessed portion forming a recess relative to the otherwise cylindrical outer surface of wall 4. Viewed in longitudinal section, the third locking means 16 has a circular cross section. Alternatively, the first locking means and/or the third locking means can have a different cross section, e. g. V-shaped, rectangular or square, or can be formed by single, multiple or circumferentially arranged projections or ridges projecting from the otherwise cylindrical outer surface 4a of the wall 4. Also, a slightly barb-like cross section is possible in order to enhance the locking or retaining effect to be described subsequently.

Fig. 2 shows a cartridge 20 filled with a medically active substance 22. Cartridge 20 has a distal end 24 closed by a closure member 26 like a septum, which can be pierced by proximal end 8b of needle 8. A proximal end 28 of the cartridge 20 is closed by a piston 30 that is slideably moveable within the cartridge. A cylindrical wall 32 encloses an internal space 34 of the cartridge 20.

Fig. 3 shows a housing 40 or syringe body of the syringe according to the invention. The housing 40 has a cylindrical wall 42, an open proximal end 44 and a nearly closed distal end 46 having an opening 48 permitting the needle 8 being distally extended there through.

A second locking means 50 is provided on an inside of the cylindrical wall 42 of the housing 40, and is configured to automatically lockingly cooperate with the first locking means 14 of the cartridge holder 2 when it is moved into its distal position. In the described embodiment, the second locking means 50 is formed as a circumferentially extending projection 52, projecting inwards relative to a cylindrical inside surface 54 of the housing 40. Viewed in a longitudinal section, the projection 52 has a circular cross section corresponding to the circular cross section of the first locking means 14. Also, the second locking means 50 may have a different cross section, e.g. V-shaped, rectangular or square, or a slightly barb-like design.

A fourth locking means 56 is provided on the inside of the cylindrical wall 42 of the housing at a distance D from the second locking means 50. The fourth locking means 56 is configured similar to the second locking means 50 and automatically and lockingly cooperates with the third locking means 16 when the cartridge holder 2 is moved into its proximal position. The distance D can be equal or greater than the distance d. As an example, (D-d) can be a distance by which the cartridge holder is moved between its distal and proximal positions, respectively, i. e. the distance between the distal and proximal positions of the cartridge holder. When the second and fourth locking means are formed by projections or dimples, they can act as a guiding and centering means for the cartridge holder. In case the distances d, D are equal, the second and fourth locking means both cooperate with the first and third locking means, respectively, when the cartridge holder is in its distal position, and the fourth and first locking means cooperate when the cartridge holder is in its proximal position.

Fig. 4 shows a plunger 60 having a plunger rod 62, a manipulating means 64 provided on a proximal end 66 of the plunger rod 62 and a first engagement means 68 provided on a distal end 70 of the plunger rod 62. The first engagement means 68 has a number of radial projection 72 projecting from an engagement body 74. The radial projections 72 are elastically moveable and/or defomiable relative to the engagement body 74. In an undeformed state, before being introduced into the internal space 34 of the cartridge 20, the first engagement means 68 has an outer diameter Da which is grater than an inner diameter Di of the cartridge 20, providing a retaining effect.

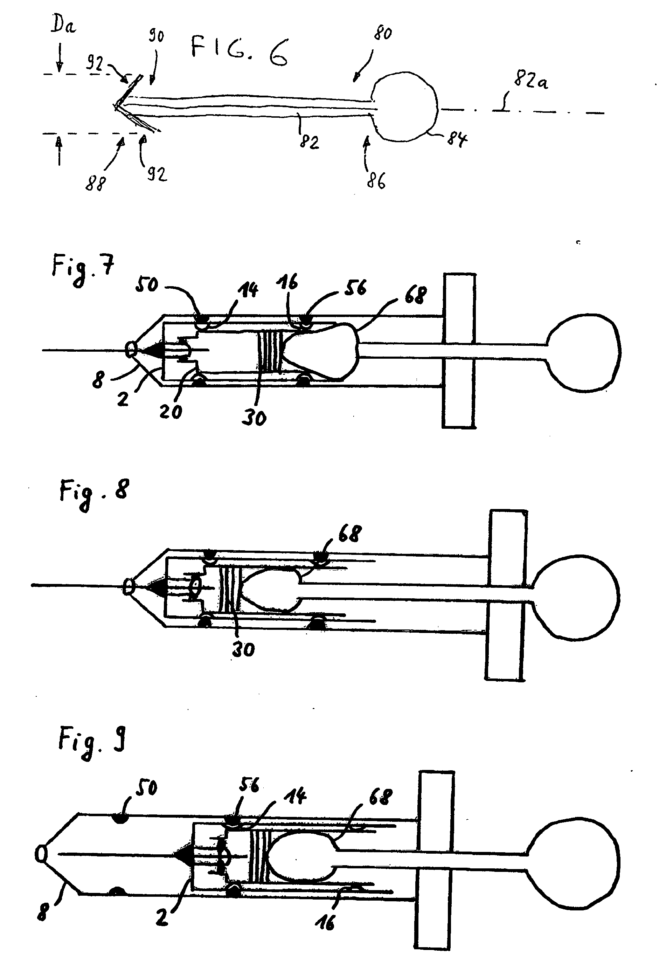

Fig. 6 shows an alternative embodiment of a plunger 80, having a plunger rod 82 provided with a manipulating means 84 on a proximal end 86 and a first engagement means 88 at a distal end 90. In this example, the first engagement means 88 takes the form of a flexible Belleville spring having at least two flexible arms 92 arranged at an angle of less than 90° relative to a longitudinal axis 82a of the plunger rod 82. Instead of several arms, the Belleville spring can be formed by a conical element. In this case the representation of Fig. 6 can be considered as a longitudinal section wherein the portions designated with 92 represent a circumferentially extending conical wall. In any case, the first engagement means 88 in an undeformed state has an outer diameter Da greater than the inner diameter Di of the cartridge 20 providing a retaining effect.

Fig. 5 shows an assembled state of a safety syringe. The cartridge 20 is received within the internal space 12 of the cartridge holder 2, and the cartridge holder 2 in turn is received within the housing or syringe body 40. The cartridge is not yet moved in its distal position within the cartridge holder so that the proximal end 8b of the needle 8 has not yet pierced the closure element 26 of the cartridge 20. Fig. 5 shows the cartridge holder 2 in the proximal position within the housing 40, in which position needle 8 is retracted inside the housing 40. In this position, the third locking means cooperates with the fourth locking means and locks the cartridge holder 2 in its position relative to the housing 40.

Fig. 5 further shows the plunger 60 introduced into the housing 40. Additionally, plunger guides 76 are shown in order to maintain a longitudinal position of the plunger, in which the longitudinal axis of the plunger rod is essentially aligned with a longitudinal axis of the housing.

When the substance 22 contained in the cartridge 20 is to be administered, the plunger is pushed distally (to the left in Fig. 5) whereby the cartridge holder is moved from its proximal position, shown in Fig. 5, to the left in a distal position in which the needle 8 projects distally out of the housing 40. In this position, the first locking means automatically lockingly cooperates with the second locking means and locks the cartridge holder in its distal position. Before, after or during such movement of the cartridge holder, the cartridge 20 is moved into its distal position relative to the cartridge holder 2, so that the proximal end 8b of the needle pierces the closure element 26 of the cartridge.

In this position, i. e. when both the cartridge holder 2 is in its distal position relative to the housing 40, and also the cartridge 20 is in its distal position relative to the cartridge holder 2, further distal movement of the plunger will move the piston 30 distally, leading to the substance 22 being ejected out of the needle 8.

After completion of the injection, when the piston 30 has been moved distally (to the left in Fig. 5), the plunger 60 is retracted in proximal direction. Due to the particular design of the engagement body 74 with the first engagement means 68, proximal movement of the plunger does not lead to proximal movement of the engagement body 74 relative to the cartridge 20, but will lead to proximal movement of the cartridge 20 together with the cartridge holder 2 relative to the housing, whereby the needle 8 is retracted into the housing.

Thus, by proximal movement or retraction of the plunger 60 after completion of an injection, the cartridge holder 2 is moved back into its proximal position shown in Fig. 5. Upon reaching its proximal position, the cartridge holder is automatically locked in this position by cooperation of the third and fourth locking means.

The design of the engagement body 74 with the first engagement means 68 (or 88 as shown in Fig. 6) enables the user to retract the cartridge holder from its distal position into its proximal position without the plunger being retracted out of the cartridge. A minimum force or pulling force required to move the first engagement means proximally relative to the cartridge is greater than a force required to overcome the locking effect of the first and second locking means, and also is greater than a force required to overcome the locking effect of the third and fourth locking means, or in other words, to move the cartridge holder proximally relative to the housing when locked in its distal position.

Fig. 7 shows the syringe ready for injection. The cartridge holder is in its distal position within the housing, and the cartridge is in its distal position within the cartridge holder. The proximal end 8b of the needle has pierced the closure element 26 of the cartridge and slightly projects into the cartridge. The engagement body 74 of the plunger 60 is ready to be pushed into the cartridge.

Fig. 7 also shows the first locking means lockingly cooperating with the second locking means, locking the cartridge holder in its distal position within the housing.

Fig. 8 shows a distal position of the piston 30, indicating completion of the injection. The engagement body 74 of the plunger 60 has reached its distal position within the cartridge 20. Upon retraction of the plunger, or movement in proximal direction, the cartridge together with the cartridge holder are moved proximally, as shown in Fig. 9. Upon reaching its proximal position, the cartridge holder 2 is automatically locked by the cooperation of the third and fourth locking means.

As mentioned, the force required to release and move the cartridge holder from its distal position (Fig. 7, Fig. 8) into its proximal position (Fig. 5, Fig. 9) is less than a minimum pulling force required to move the engagement body 74 proximally within the cartridge. This design feature allows retracting of the cartridge holder without proximal movement of the engagement body 74 with the cartridge, or retraction of the plunger out of the syringe body.

The cartridge is received in the cartridge holder by a friction fit and is then retained in its distal position relation to the cartridge holder by friction so that a pulling force on the plunger is transmitted to the cartridge holder without moving the cartridge proximally relative to the cartridge holder.

### Reference numerals

| | |
|---|---|
| 2 | cartridge holder |
| 4 | cylindrical wall |
| 4a | outer surface |
| 6 | distal end |
| 8 | needle |
| 8a | distal end |
| 8b | proximal end |
| 10 | proximal end |
| 12 | internal space |
| 14 | first locking means |
| 16 | third locking means |
| 20 | cartridge |
| 22 | substance |
| 24 | distal end |
| 26 | closure element |
| 28 | proximal end |
| 30 | piston |
| 32 | cylindrical wall |
| 34 | internal space |
| 40 | housing (syringe body) |
| 42 | cylindrical wall |
| 44 | proximal end |
| 46 | distal end |
| 48 | opening |
| 50 | second locking means |
| 52 | projection |
| 54 | inside surface |
| 56 | fourth locking means |
| 60 | plunger |
| 62 | plunger rod |
| 64 | manipulating means |
| 66 | proximal end |
| 68 | first engagement means |
| 70 | distal end |
| 72 | radial projection |
| 74 | engagement body |
| 76 | plunger guide |
| 80 | plunger |
| 82 | plunger rod |
| 82a | longitudinal axis |
| 84 | manipulating means |
| 86 | proximal end |
| 88 | second engagement means |
| 90 | distal end |
| 92 | arm |
| | |
| | |
| d | distance 14, 16 |
| D | distance 50, 56 |
| Da | outer diameter of 68, 88 |
| Di | inner diameter of 20 |

## Claims

1. Safety syringe, comprising:
a housing (40) having an open proximal end (44) for introducing a cartridge holder (2), the housing (40) further having a distal end (46) provided with an opening (48) for extending a needle out of the housing (40);
a cartridge holder (2) slidably mounted within the housing (40), the cartridge holder (2) having a distal end (6) mounting a needle (8) and an open proximal end (10) for receiving a cartridge, the cartridge holder (2) being movable between a distal position, in which the needle (8) projects out of the distal end (46) of the housing (40), and a proximal position, in which the needle (8) is retracted into the housing (40), a first piercing tip (8a) of the needle (8) projecting distally from the cartridge holder (2) and a second piercing tip (8b) of the needle (8) projecting proximally inside the cartridge holder (2);
a cartridge (20) received in the cartridge holder (2), the cartridge (20) being filled with a medically active substance (22) and being movable in the cartridge holder(2) between a distal position and a proximal position, the cartridge being closed at a distal end (24) by a closure member(26), the closure member (26) being piercable by the second piercing tip (8b) of the needle (8) upon movement of the cartridge (20) into the distal position of the cartridge (20), the cartridge (20) being closed at a proximal end (28) by a piston (30) slidably movable within the cartridge (20);
a plunger rod (62) extending into the housing (40) through the open end (44) of the housing (40),
**characterized by** the plunger rod (62) having at a distal end (70) a first engagement means (68) for engaging an inside surface (54) of the cartridge (20), the first engagement means (68) 2being designed to be moved proximally relative to the cartridge (20) only upon a minimum pulling force being exerted on the plunger rod (62);
a first locking means (14) on the cartridge holder (2) and a second locking means (50) inside the housing (40), the first and second locking means (14, 50) cooperatingly and automatically locking the cartridge holder (2) upon movement of the cartridge holder (2) in its distal position,
the first and second locking means (14, 50) being designed to allow the cartridge holder (2) to be moved from its distal position into its proximal position upon exertion of a proximally directed pulling force on the plunger rod (62) which is less than the minimum pulling force,
the cartridge (20) being received in the cartridge holder (2) by a friction fit and is then retained in its distal position relation to the cartridge holder (2) by friction so that a pulling force on the plunger rod (62) is transmitted to the cartridge holder (2) without moving the cartridge (20) proximally relative to the cartridge holder (2).

2. Safety syringe as claimed in claim 1, wherein the plunger rod (62) at its distal end is provided with a second engagement means (88) for cooperating with the piston (30), the second engagement means (88) enabling the plunger rod (62) to pull on the piston (30) into a proximal direction for aspiration.

3. Safety syringe as claimed in claim 1 or 2, wherein inside the housing (40) a fourth locking means (56) and on the cartridge holder (2) a third locking means (16) are provided, the fourth locking means (56) and the third locking means (16) cooperatingly and automatically locking the cartridge holder (2) upon movement of the cartridge holder (2) in its proximal position.

4. Safety syringe as claimed in claim 1 or 2, wherein inside the housing (40) a fourth locking means (56) is provided, the fourth locking (56) means and the first locking means (14) cooperatingly and automatically locking the cartridge holder (2) upon movement of the cartridge holder (2) into its proximal position.

5. Safety syringe as claimed in any of claims 1 to 4, wherein the first engagement means (68) comprises a barbed design.

6. Safety syringe as claimed in any of claims 1 to 5, wherein the second engagement means (88) comprises a screw thread.

7. Safety syringe as claimed in any of the claims 1 to 6, wherein the second engagement means (88) comprises a hook.

## Patentansprüche

1. Sicherheitsspritze, umfassend:
ein Gehäuse (40) mit einem offenen proximalen Ende (44) zum Einsetzen eines Patronenhalters (2), wobei das Gehäuse (40) weiterhin ein distales Ende (46) aufweist, das mit einer Öffnung (48) versehen ist, um eine Nadel aus dem Gehäuse (40) zu führen;
einen Patronenhalter (2), der verschieblich innerhalb des Gehäuses (40) gehalten ist, wobei der Patronenhalter (2) ein distales Ende (6), das eine Nadel (8) hält, und ein offenes proximales Ende (10) zum Aufnehmen einer Patrone aufweist, wobei der Patronenhalter (2) zwischen einer distalen Stellung, in der die Nadel (8) aus dem distalen Ende (46) des Gehäuse (40) vorsteht, und einer proximalen Stellung, in der die Nadel (8) in das Gehäuse (40) zurückgezogen ist, bewegbar ist, wobei eine erste Einstechspitze (8a) der Nadel (8) distal aus dem Patronenhalter (2) vorsteht und eine zweite Einstechspitze (8b) der Nadel (8) proximal in den Patronenhalter (2) vorsteht;
eine Patrone (20), die in dem Patronenhalter (2) aufgenommen ist, wobei die Patrone (20) mit einer medizinisch aktiven Substanz (22) gefüllt ist, und in dem Patronenhalter (2) zwischen einer distalen Stellung und einer proximalen Stellung bewegbar ist, wobei die Patrone an einem distalen Ende (24) durch ein Verschlusselement (26) verschlossen ist, wobei das Verschlusselement (26) durch die zweite Einstechspitze (8b) der Nadel (8) bei einer Bewegung der Patrone (20) in die distale Stellung der Patrone (20) durchstochen werden kann, wobei die Patrone (20) an einem proximalen Ende (28) durch einen Kolben (30) verschlossen ist, der innerhalb der Patrone (20) gleitend verschieblich ist;
eine Kolbenstange (62), die sich durch das offene Ende (44) des Gehäuse (40) in das Gehäuse (40) erstreckt,
**dadurch gekennzeichnet, dass** die Kolbenstange (62) an einem distalen Ende (70) ein erstes Eingreifmittel (68) zum Zusammenwirken mit einer inneren Oberfläche (54) der Patrone (20) aufweist, wobei das erste Eingreifmittel (68) ausgelegt ist, um nur dann in proximaler Richtung relativ zu der Patrone (20) bewegt zu werden, wenn eine minimale Zugkraft auf die Kolbenstange (62) ausgeübt wird,
ein erstes Verriegelungsmittel (14) auf dem Patronenhalter (2) und ein zweites Verriegelungsmittel (50) innerhalb des Gehäuses (40), wobei die ersten und zweiten Verriegelungsmittel (14, 50) zusammenwirkend und automatisch den Patronenhalter (2) bei einer Bewegung des Patronenhalters (2) in seine distale Stellung verriegeln,
wobei die ersten und zweiten Verriegelungsmittel (14, 50) ausgelegt sind, um dem Patronenhalter (2) zu ermöglichen, von seiner distalen Stellung in seine proximale Stellung bewegt zu werden, bei Ausübung einer in proximaler Richtung gerichteten Zugkraft auf die Kolbenstange (62), die kleiner ist als die minimale Zugkraft,
wobei die Patrone (20) in dem Patronenhalter (2) mittels eines reibungsbehafteten Sitzes aufgenommen ist und dann in ihrer distalen Stellung relativ zu dem Patronenhalter (2) durch Reibung festgehalten ist, so dass eine auf die Kolbenstange (62) ausgeübte Zugkraft auf den Patronenhalter (2) übertragen wird, ohne die Patrone (20) relativ zu dem Patronenhalter (2) in proximaler Richtung zu bewegen.

2. Sicherheitsspritze nach Anspruch 1, **dadurch gekennzeichnet, dass** die Kolbenstange (62) an ihrem distalen Ende mit einem zweiten Eingreifmittel (88) zum Zusammenwirken mit dem Kolben (30) versehen ist, wobei das zweite Eingreifmittel (88) die Kolbenstange (62) in die Lage versetzt, zum Ansaugen an dem Kolben (30) in einer proximalen Richtung zu ziehen.

3. Sicherheitsspritze nach Anspruch 1 oder 2, **dadurch gekennzeichnet, dass** innerhalb des Gehäuses (40) ein viertes Verriegelungsmittel (56) und auf dem Patronenhalter (2) ein drittes Verriegelungsmittel (16) vorgesehen sind, wobei das vierte Verriegelungsmittel (56) und das dritte Verriegelungsmittel (16) zusammenwirkend und automatisch den Patronenhalter (2) bei einer Bewegung des Patronenhalters (2) in seine proximale Stellung verriegeln.

4. Sicherheitsspritze nach Anspruch 1 oder 2, **dadurch gekennzeichnet, dass** innerhalb des Gehäuses (40) ein viertes Verriegelungsmittel (56) vorgesehen ist, wobei das vierte Verriegelungsmittel (56) und das erste Verriegelungsmittel (14) zusammenwirkend und automatisch den Patronenhalter (2) bei einer Bewegung des Patronenhalters (2) in seine proximale Stellung verriegeln.

5. Sicherheitsspritze nach einem der Ansprüche 1 bis 4, **dadurch gekennzeichnet, dass** das erste Eingreifmittel (68) eine widerhakenartige Ausführung umfasst.

6. Sicherheitsspritze nach einem der Ansprüche 1 bis 5, **dadurch gekennzeichnet, dass** das zweite Eingreifmittel (88) ein Schraubgewinde umfasst.

7. Sicherheitsspritze nach einem der Ansprüche 1 bis 6, **dadurch gekennzeichnet, dass** das zweite Eingreifmittel (88) einen Hakten umfasst.

## Revendications

1. Seringue de sécurité, comprenant :
un boîtier (40) ayant une extrémité proximale ouverte (44) pour introduire un porte-cartouche (2), le boîtier (40) ayant en outre une extrémité distale (46) pourvue d'une ouverture (48) pour faire sortir une aiguille du boîtier (40);
un porte-cartouche (2) monté dans le boîtier (40) d'une manière coulissante, le porte-cartouche (2) ayant une extrémité distale (6) sur laquelle une aiguille (8) est montée et une extrémité proximale ouverte (10) pour recevoir une cartouche, le porte-cartouche (2) étant amovible entre une position distale, dans laquelle l'aiguille (8) se projette hors de l'extrémité distale (46) du boîtier (40), et une position proximale, dans laquelle l'aiguille (8) est rétractée dans le boîtier (40), une première pointe perçante (8a) de l'aiguille (8) faisant saillie du porte-cartouche d'une manière distale (2) et une deuxième pointe perçante (8b) de l'aiguille (8) faisant saillie à l'intérieur du porte-cartouche (2) d'une manière proximale;
une cartouche (20) reçue dans le porte-cartouche (2), la cartouche (20) étant remplie d'une substance médicallement active (22) et étant amovible dans le porte-cartouche (2) entre une position distale et une position proximale, la cartouche étant fermée à une extrémité distale (24) par un membre de fermeture (26), le membre de fermeture (26) pouvant être percé par la deuxième pointe perçante (8b) de l'aiguille (8) lors du déplacement de la cartouche (20) dans la position distale de la cartouche (20), la cartouche (20) étant fermée à une extrémité proximale (28) par un piston (30) amovible d'une manière coulissante dans la cartouche (20);
une tige de plongeur (62) s'étendant dans le boîtier (40) à travers l'extrémité ouverte (44) du boîtier (40),
**caractérisée par** la tige de plongeur (62) ayant à une extrémité distale (70) un premier moyen d'engagement (68) pour engager une surface intérieure (54) de la cartouche (20), le premier moyen d'engagement (68) étant conçu pour être déplacé d'une manière proximale par rapport à la cartouche (20) seulement lorsqu'une force de traction minimale est exercée sur la tige de plongeur (62);
un premier moyen de verrouillage (14) sur le porte-cartouche (2) et un deuxième moyen de verrouillage (50) à l'intérieur du boîtier (40), le premier et le deuxième moyen de verrouillage (14, 50) verrouillant d'une manière concourante et automatiquement le porte-cartouche (2) lors du déplacement du porte-cartouche (2) dans sa position distale,
le premier et le deuxième moyen de verrouillage (14, 50) étant conçus pour permettre au porte-cartouche (2) d'être déplacé de sa position distale à sa position proximale lors de l'emploi d'une force de traction dirigée dans le sens proximal sur la tige de plongeur (62) qui est moindre que la force de traction minimale;
la cartouche (20) étant reçue dans le porte-cartouche (2) par ajustement serré et puis elle est retenue dans sa position distale par rapport au porte-cartouche (2) par frottement de telle sorte qu'une force de traction sur la tige de plongeur (62) est transmise au porte-cartouche (2) sans déplacer la cartouche (20) d'une manière proximale par rapport au porte-cartouche (2).

2. Seringue de sécurité selon la revendication 1, dans laquelle la tige de plongeur (62) est pourvue, à son extrémité distale, d'un deuxième moyen d'engagement (88) pour coopérer avec le piston (30), le deuxième moyen d'engagement (88) permettant à la tige de plongeur (62) de tirer sur le piston (30) dans une direction proximale pour aspiration.

3. Seringue de sécurité selon la revendication 1 ou 2, dans laquelle un quatrième moyen de verrouillage (56) est fourni à l'intérieur du boîtier (40) et un troisième moyen de verrouillage (16) est fourni sur le porte-cartouche (2), le quatrième moyen de verrouillage (56) et le troisième moyen de verrouillage (16) verrouillant d'une manière concourante et automatiquement le porte-cartouche (2) lors du déplacement du porte-cartouche (2) dans sa position proximale.

4. Seringue de sécurité selon la revendication 1 ou 2, dans laquelle un quatrième moyen de verrouillage (56) est fourni à l'intérieur du boîtier (40), le quatrième moyen de verrouillage (56) et le premier moyen de verrouillage (14) verrouillant d'une manière concourante et automatiquement le porte-cartouche (2) lors du déplacement du porte-cartouche (2) dans sa position proximale.

5. Seringue de sécurité selon l'une quelconque des revendications 1-4, dans laquelle le premier moyen d'engagement (68) comprend une conception à barbillon.

6. Seringue de sécurité selon l'une quelconque des revendications 1-5, dans laquelle le deuxième moyen d'engagement (88) comprend un filetage de vis.

7. Seringue de sécurité selon l'une quelconque des revendications 1-6, dans laquelle le deuxième moyen d'engagement (88) comprend un crochet.
